# EUROPÄISCHE PATENTSCHRIFT

(11) **EP 0 537 429 B1**
(45) Veröffentlichungstag und Bekanntmachung des Hinweises auf die Patenterteilung: **12.06.1996**
(21) Anmeldenummer: 92113088.6
(22) Anmeldetag: 31.07.1992
(51) Int. Cl.: C08H 5/00, A61K 35/10, C02F 1/56

(54) **Modifizierte Huminate, Verfahren zu ihrer Herstellung und Verwendung**
Modified huminates, process for obtaining the same and their uses
Huminates modifiés, procédé pour leur préparation et applications

(30) Priorität: 17.10.1991 DE 4134384
(43) Veröffentlichungstag der Anmeldung: 21.04.1993
(73) Patentinhaber: RÜTGERSWERKE AKTIENGESELLSCHAFT, 60326 Frankfurt (DE)
(72) Erfinder: Riede, Urs N., Prof. Dr., W-7800 Freiburg (DE); Seubert, Bernhard, W-6803 Edingen (DE)

(56) Entgegenhaltungen:
- EP-A- 0 362 471
- ANALYTICAL CHEMISTRY SYMPOSIA SERIES; CHROMATOGRAPHIY AND MASS SPECTROMETRY IN BIOMEDICAL SCIENCES, 2 Bd. 14, 1983, Seiten 149 - 157 A. MARCHESINI ET AL. 'Isolation and characterization of natural polymers extracted from soil and compost by ion-exchange thin layer chromatography'
- ADVANCES IN ORGANIC GEOCHEMISTRY Bd. 16, Nr. 4-6, 1989, UK Seiten 853 - 864 L. S. K. PANG ET AL. 'Chemistry of alkali extraction of brown coals - I. kinetics, characterisation and implications to coalification.
- MIKROCHIMICA ACTA Bd. 2, Nr. 1-2, 1983, WIEN Seiten 151 - 157 S. M. TZOUWARA- KARAYANNI ET AL. 'Kinetic study of the chemical behavior of quercetin and kaempferol in alkaline media'

## Beschreibung

Die Erfindung betrifft ein Verfahren zur Herstellung modifizierter synthetischer Alkali- oder Ammoniumhuminate. Aus EP-A-0 281 678 ist ein Verfahren zur synthetischen Herstellung von niedermolekularen Alkalihuminaten durch Oxidation mehrwertiger Phenole in schwach alkalischem Medium bekannt.

Diese Huminate sind gering toxisch, wirken aber heilend und detoxifizierend in einem weiten Wirkungsbereich, wie dies auch in den folgenden Patentanmeldungen beansprucht ist: DE-A 38 30 616, DE-A 40 22 795, DE-A-41 05 395 und DE-A-41 29 396.

Allerdings ist die spezifische Wirksamkeit relativ gering, so daß die Aufgabe bestand, ähnliche, gering toxische Stoffe bereitzustellen, die eine deutlich höhere Wirksamkeit bei gleich großem Wirkungsspektrum haben.

Die Lösung der Aufgabe erfolgt durch ein Verfahren zur Herstellung von Alkali- oder Ammoniumhuminaten gemäß der Ansprüche 1 - 9 sowie die Verwendung dieser Huminate gemäß der Ansprüche 11 bis 17.

Es wurde gefunden, daß im alkalischen wäßrigen Milieu durch Oxidation von Verbindungen der allgemeinen Formeln, 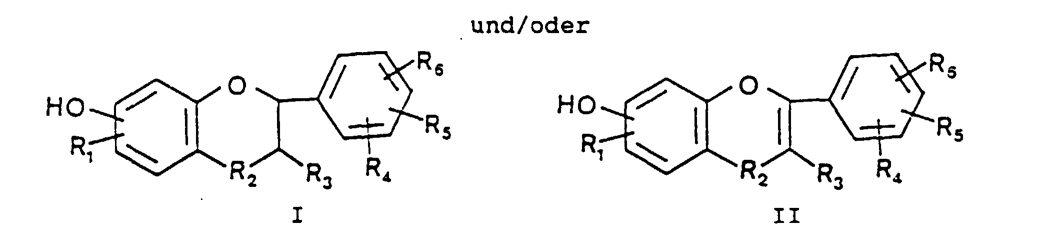 wobei R₁, R₃ und R₄ gleich oder verschieden sind und eine OH-Gruppe oder Wasserstoff darstellen, R₂ eine CO- oder CH2-Gruppe bedeutet und R₅ und R₆ gleich oder verschieden sind und Wasserstoff, eine OH- oder Methoxygruppe darstellen, wobei R₄, R₅ und R₆ nicht alle gleichzeitig Wasserstoff sind, hergestellte huminstoffartige modifizierte Alkali- oder Ammoniumhuminate nicht toxisch sind und das gleiche Wirkungsspektrum wie die aus EP-B-0 281 679 bekannten, natürlichen und die aus EP-B-0 281 676 bekannten, synthetisch hergestellten, niedermolekularen Alkalihuminate zeigen.

Im Gegensatz zu diesen sind die erfindungsgemäß hergestellten modifizierten Huminate bis zu 100mal wirksamer als die nicht modifizierten, niedermolekularen Alkalihuminate.

Die modifizierten Alkali- oder Ammoniumhuminate sind entweder ausschließlich oder teilweise aufgebaut aus Molekülen gemäß der Formeln I und/oder II in der oben angegebenen Bedeutung, wobei die möglichen anderen Bausteine mehrwertige Phenole sind.

Beispiel für Moleküle der allgemeinen Formeln I oder II sind:
Narigenin
Eriodictyol
Hesperitin
Pelargonidin
Cyanidin Delphinidin
Päonidin
Syringidin
Catechin bzw.
Epicatechin
Gallocatechin
Apigenin
Fisetin Robinetin
Gossypetin
Luteolin
Kämpferol
Quercetin
Morin oder Myricetin

Bevorzugte Bausteine sind Quercetin und Catechin.

Gegenstand der Erfindung ist ein verfahren zur Herstellung von Alkali- oder Ammoniumhuminaten, **dadurch gekennzeichnet**, daß alkalische oder ammoniakalische, wäßrige Lösungen, die Verbindungen der allgemeinen Formeln 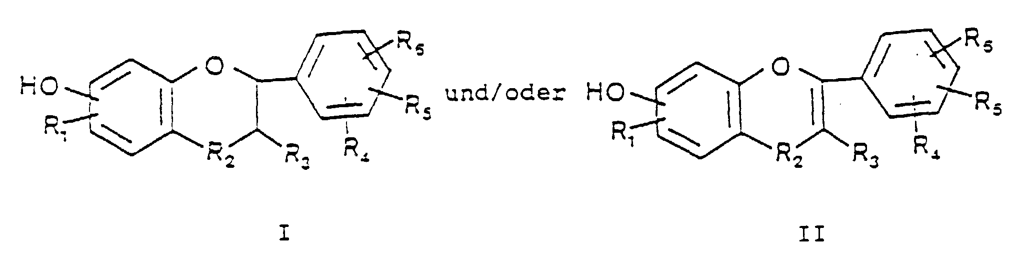 enthalten, wobei R₁, R₃ und R₄ gleich oder verschieden sind und eine OH-Gruppe oder Wasserstoff darstellt, R₂ eine CO- oder CH₂-Gruppe bedeutet und R₅ und R₆ gleich oder verschieden sind und Wasserstoff, eine OH- oder Methoxygruppe darstellen, wobei R₄,R₅ und R₆ nicht alle gleichzeitig Wasserstoff sind, bei einer Temperatur im Bereich von 15 bis 40°C oxidiert werden, danach die Reaktionsmischung auf einen pH-Wert im Bereich von 4,5 bis 7,0 eingestellt und/oder gepuffert wird und die Alkali- oder Ammoniumhuminate mit einem Molekulargewicht bis zu 30 000 D durch präparative chromatographiemethoden, Ultrafiltration, Ultrazentrifugation oder Elektrodialyse gereinigt und von unerwünschten Nebenprodukten abgetrennt werden, sowie die Huminate, hergestellt nach diesem Verfahren und ihre Verwendungen gemäß der Ansprüche 11 bis 17.

Die Herstellung der Alkali- oder Ammoniumhuminate erfolgt, indem alkalische, wäßrige Lösungen, (pH > 9) die Verbindungen der allgemeinen Formeln I und/oder II enthalten, wobei die Formeln I und II die oben angegebene Struktur und Bedeutung haben, bei einer Temperatur im Bereich von 15 bis 40°C oxidiert werden, danach die Reaktionsmischung auf einen pH-Wert im neutralen oder schwach sauren Bereich von (4,5 bis 7, insbesondere 5,5 bis 6,5) eingestellt und/oder gepuffert wird und die Huminate durch präparative Chromatographiemethoden, Ultrafiltration, Ultrazentrifugation oder Elektrodialyse gereinigt und von unerwünschten Nebenprodukten abgetrennt werden.

Die Verbindungen gemäß der Formeln I und II können als einzelne Reinsubstanzen sowie auch im beliebigen Gemisch miteinander eingesetzt werden. Sie können im Reaktionsgemisch auch mit mehrwertigen Phenolen eingesetzt werden, was aus wirtschaftlichen Gründen durchaus angebracht sein kann.

Da die entstehenden Huminate aus Verbindungen der Formeln I und/oder II und aus mehrwertigen Phenolen mit steigendem Anteil an mehrwertigen Phenolen eine verminderte physiologische Wirksamkeit zeigen, ist das bevorzugte molare Verhältnis der Verbindungen der allgemeinen Formeln I und/oder II zu mehrwertigen Phenolen im Bereich von 1 : 0 bis 1 : 10.

Es ist einleuchtend, daß die eingesetzten Verbindungen möglichst frei von sonstigen Nebenprodukten sein sollen, denn dadurch werden unerwünschte Nebenreaktionen vermieden bzw. die erhaltenen Endprodukte lassen sich ohne weitere chemische Aufarbeitung - die auch mit unerwünschten chemischen Veränderungen der Endprodukte verbunden wären - erhalten.

Zur Umsetzung werden die Verbindungen der Formeln I und/oder II sowie gegebenenfalls die mehrwertigen Phenole in einer wäßrigen Alkalilauge oder Ammoniumlösung gelöst. Als Alkalilauge können alle Alkalimetallhydroxide verwendet werden. Aus wirtschaftlichen Gründen sind Natrium- und Kaliumhydroxid bevorzugt. Die Menge des eingesetzten Ammoniaks oder Alkalihydroxids liegt bevorzugt im Bereich der 1,8 bis 2,0-fach stöchiometrischen Menge, die zur Neutralisation aller phenolischen OH-Gruppen notwendig ist. Dabei stellt sich ein pH-Wert der Reaktionslösung von 9 bis 12 ein. Allerdings ist auch ein höherer pH-Wert (bis etwa 14) nicht störend.

Im allgemeinen ist es ausreichend, wenn als Wasser ein entmineralisiertes Wasser mit einer Leitfähigkeit von 6 bis 10 µS/cm und einem pH-Wert im Bereich von 5 bis 7 eingesetzt wird, als Alkalihydroxid entweder die Qualität "chemisch rein" oder der Reinheitsgrad nach DAB 9.

Die Oxidation der Einsatzprodukte in alkalischer Lösung kann entweder elektrochemisch oder plasmachemisch bzw. chemisch durch Über- oder Durchleiten von Sauerstoff oder eines sauerstoffhaltigen Gasgemisches erfolgen.

Die elektrochemische Oxidation erfolgt in einem elektrochemischen Reaktor, wobei die Oxidationsgeschwindigkeit durch Einstellung der Anodenspannung und der Stromdichte bestimmt wird.

Die Anodenspannung kann im Bereich von 4 bis 15 V und die Stromdichte im Bereich von 0,5 bis 4 A/cm² variiert werden. Dabei liegt die Reaktionszeit im Bereich von 1 bis 3 d.

Die plasmachemische Oxidation erfolgt in einer an sich bekannten Apparatur zur Koronaentladung, wobei die Oxidationsgeschwindigkeit durch Einstellung der Betriebsspannung und der Feldstärke bestimmt wird. Die Spannung kann im Bereich von 20 bis 250 kV bei Frequenzen von 16 2/3 bis 400 Hz und die Feldstärke von 80 kV/cm bis 200 kV/cm variiert werden. Dabei liegt die Reaktionszeit im Bereich von 15 bis 120 Minuten.

Zur chemischen Oxidation wird die alkalische Lösung der Verbindungen gemäß der Formeln I und/oder II in ein Reaktionsgefäß gebracht, das den unkontrollierten Zutritt von Luft verhindert, das heißt in ein geschlossenes Reaktionsgefäß, das aber mit einer Vorrichtung versehen ist, mittels der Gase ein- bzw. durchgeleitet werden können.

Nun wird unter Rühren Sauerstoff oder ein sauerstoffhaltiges Gasgemisch in ständigem Gasstrom entweder über oder durch die Reaktionslösung oder unter Druck auf die Reaktionslösung geleitet, wobei die Temperatur des Reaktionsgemisches im Bereich von 10 bis 40°C, bevorzugt im Bereich von Raumtemperatur bis 30°C liegt. Als sauerstoffhaltiges Gasgemisch kann auch Luft eingesetzt werden, die aber vorher zur Adsorption von CO₂ und zur Entfernung von Staubpartikeln über einen alkalischen Filter geleitet werden muß.

Diese Oxidationsreaktion dauert, je nach gewählter Temperatur und Intensität der Sauerstoffzufuhr 5 bis 20 Tage. Während dieser Reaktionszeit färbt sich die Lösung intensiv dunkelbraun.

Die chemische Oxidation kann auch durch Reaktion mit milden Oxidationsmitteln wie Wasserstoffperoxid, seinen Anlagerungsverbindungen oder Persulfaten erfolgen.

Es ist ein Vorteil des erfindungsgemäßen Verfahrens, daß bei Einsatz von Verbindungen der allgemeinen Formeln I und/oder II im schwach alkalischen Medium sowohl bei der elektrochemischen Oxidation als auch bei der plasmachemischen und der chemischen Oxidation die Bildungsreaktion der modifizierten Huminstoffe nicht zu hochmolekularen Produkten führt, sondern bei Produkten mit einem Molekulargewicht von bis zu 30 000 D von selbst zum Stillstand kommt. Dadurch erübrigt sich eine ständige Kontrolle über den Verlauf der Oxidationsreaktion und es ist nicht nachteilig, wenn die Reaktionsdauer länger ist, als es notwendig wäre.

Die aus der chemischen, plasmachemischen oder der elektrochemischen Oxidationsreaktion resultierende dunkelbraune Lösung wird nun neutralisiert und auf einen pH-Wert im Bereich von 4,5 bis 7,0, bevorzugt von 5,5 bis 6,5 eingestellt und gepuffert. Dies erfolgt entweder durch Zugabe von Säure oder durch Einwirkung von saurem Ionenaustauscher und/oder nachfolgender Zugabe einer entsprechenden Pufferlösung (z. B. Phosphat-, Tris- oder Zitronensäure- Puffer).

Sofern die neutralisierte und gepufferte Lösung unerwünschte Schwebstoffe enthält, wird sie nun durch ein Trennverfahren wie Zentrifugieren (10 000 bis 30 000xg) oder Filtrieren durch ein sehr feinporiges Filtermaterial von diesen Schwebstoffen befreit. Obwohl diese Lösung für viele Anwendungszwecke direkt eingesetzt werden kann, sollte sie bei Verwendung der Produkte als Heilmittel durch an sich bekannte Reinigungsverfahren wie präparative Chromatographiemethoden, Ultrafiltration, Ultrazentrifugation oder Elektrodialyse gereinigt und von unerwünschten Nebenprodukten befreit werden.

Erhalten wird eine Lösung, die etwa 3 bis 5 % Huminate mit einem Molekulargewicht bis zu 30 000 D enthält. Die Lösung zeigt keinen Tyndall-Effekt und fluoresziert nicht. Diese Lösung kann direkt verwendet werden oder durch vorsichtiges Entfernen von Wasser, etwa mittels Gefriertrocknung zu einer 20 %igen Lösung aufkonzentriert werden.

Die erhaltene und die aufkonzentrierte Lösung ist stabil.

Bei Stabilitätskontrollen ist nach 60 Tagen Wechselbelastung 56/4°C im 12/12 h Rhythmus keine Veränderung der Parameter Gehalt, pH-Wert, Redoxspannung und Mikrodialysetest über die Zufallsschwankungen hinaus feststellbar. Die erhaltenen Huminate zeigen eine äußerst geringe Toxizität, aber eine hohe physiologische und detoxifizierende Wirksamkeit.

So ist z. B. die wundheilende Wirkung bis um das hundertfache besser als die der natürlichen und synthetischen Huminstoffe.

Die erfindungsgemäßen niedermolekularen Huminate eignen sich daher für alle Anwendungen, die von den natürlichen oder synthetischen niedermolekularen Huminstoffen bekannt sind.

Insbesondere eignen sie sich zur Verwendung als Wundheilmittel, zur Herstellung von Mitteln zur Wundheilung, zur Herstellung von hochwirksamen Moorbädern, von Nasenarzneien gegen Pollenallergien, von Antischuppenshampoos sowie von Mitteln zur Behandlung von Fischen, insbesondere von gestreßten Fischen. Darüber hinaus eignen sich die modifizierten Huminate zur Detoxifizierung von Wasser sowie von Flächen oder festen Gegenständen.

### BEISPIELE:

### Beispiel 1

29 g (0,1 Mol) Catechin (2-3,4-Dihydroxyphenyl)-chroman-3,5,7-triol) werden bei Raumtemperatur in 1000 ml einer wäßrigen 0,8 molaren Natronlaugelösung gelöst.

Die so erhaltene Lösung (pH 10) wird in einen mit Rührer, Thermometer, Gaseinführungs- und Überdruckentlastungs-Vorrichtung versehenen Glasreaktor gebracht. Danach wird bei Raumtemperatur durch die Lösung in ständigem Gasstrom Luft geleitet, die zuvor in einer Gaswaschflasche mit NaOH-Lösung von CO₂ und Staubpartikeln gereinigt wurde.

Der Gasstrom wird so reguliert, daß sich eine Temperatur der Reaktionslösung von 30 °C einstellt.

Nach einer Reaktionszeit von 10 Tagen, wenn trotz weiterer Luftzufuhr die Temperatur der Reaktionslösung absinkt, ist die Umsetzung beendet. Die entstandene dunkelbraune Lösung wird durch Zugabe von verdünnter Essigsäure auf eine pH-Wert von 6,0 eingestellt und danach mittels einer Ultrafiltration gereinigt.

Die gereinigte Lösung enthält 4,2 % eines Huminats mit einem mittleren Molekulargewicht von 2 500 D bei einer Streubreite von 1 000 bis 30 000 D. Die Lösung ist dunkelbraun, fluoresziert nicht und zeigt keinen Tyndall-Effekt.

### Beispiel 2

An 2 x 10 haarlosen Mäusen werden mit einem Mikrodermatom oberflächliche Wunden, die nur die obersten Epithelschichten erfaßten, in einer Größe von etwa 50 mm² beigebracht. Bei zehn dieser Mäuse wird die Wunde mit einer 0,02 %igen Lösung des Huminats gemäß Beispiel 1 einmal benetzt. Die anderen Mäuse bleiben unbehandelt. Während des Beobachtungszeitraumes von 7 d läßt sich folgendes beobachten:

Gegenüber den unbehandelten Mäusen nimmt bei den behandelten Versuchstieren
- die Wundfläche rascher ab,
- die Wunde trocknet früher ab,
- die Granulation setzt früher ein und
- die Wunde reinigt sich früher.

Insgesamt ist die Abheilung 2 bis 3 d früher als bei den Kontrolltieren zu beobachten.

### Beispiel 3

Eine nach Thrypsinbehandlung in Suspension gebrachte Kultur von L-Zellen (Mäusefibroblasten) wird mit 5 ppm Huminat aus Beispiel 1 versetzt.

Die Kultur wird bei 37 °C unter Verwendung eines handelsüblichen Nährmediums 48 h lang bebrütet.

Parallel hierzu wird als Vergleichsversuch eine analoge Kultur ohne Alkalihuminat gleichermaßen bebrütet.

Danach wird die Anzahl der lebenden Zellen in beiden Kulturen bestimmt. In der mit modifizierten Huminaten versetzten Kultur liegt die Zahl der lebenden Zellen um 40 % höher als in der Vergleichskultur.

### Beispiel 4

Bei Injektionen einer 1 %igen Lösung des Huminats aus Beispiel 1 an Versuchstieren (Mäuse) werden folgende Werte der LD₅₀ erhalten.

| | Fraktion (1) |
|---|---|
| s.c. | 1780 (mg/kg) |
| i.p. | 1210 (mg/kg) |
| i.V. | 890 (mg/kg) |

## Patentansprüche

1. Verfahren zur Herstellung von Alkali- oder Ammoniumhuminaten, **dadurch gekennzeichnet**, daß alkalische oder ammoniakalische, wäßrige Lösungen, die Verbindungen der allgemeinen Formeln 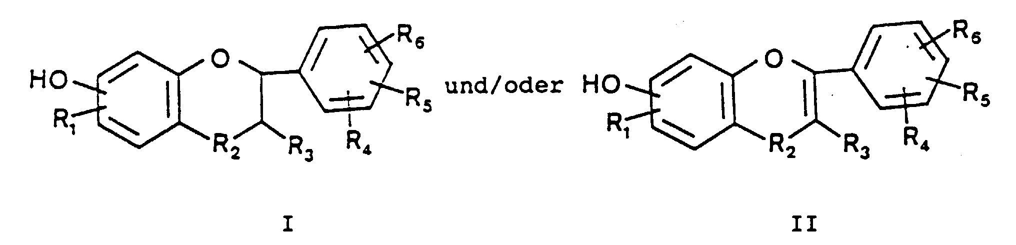 enthalten, wobei R₁, R₃ und R₄ gleich oder verschieden sind und eine OH-Gruppe oder Wasserstoff darstellt, R₂ eine CO- oder CH₂-Gruppe bedeutet und R₅ und R₆ gleich oder verschieden sind und Wasserstoff, eine OH- oder Methoxygruppe darstellen, wobei R₄,R₅ und R₆ nicht alle gleichzeitig Wasserstoff sind, bei einer Temperatur im Bereich von 15 bis 40°C oxidiert werden, danach die Reaktionsmischung auf einen pH-Wert im Bereich von 4,5 bis 7,0 eingestellt und/oder gepuffert wird und die Alkali- oder Ammoniumhuminate mit einem Molekulargewicht bis zu 30 000 D durch präparative chromatographiemethoden, Ultrafiltration, Ultrazentrifugation oder Elektrodialyse gereinigt und von unerwünschten Nebenprodukten abgetrennt werden.

2. Verfahren nach Anspruch 1, **dadurch gekennzeichnet**, daß Verbindungen der allgemeinen Formeln I und/oder II als Reinsubstanzen oder im beliebigen Gemisch miteinander eingesetzt werden.

3. Verfahren nach den Ansprüchen 1 und 2, **dadurch gekennzeichnet**, daß Verbindungen der allgemeinen Formeln I und/oder II im Gemisch mit mehrwertigen Phenolen eingesetzt werden.

4. Verfaren nach Anspruch 3, **dadurch gekennzeichnet**, daß das molare Verhältnis der Verbindungen der allgemeinen Formeln I und/oder II zu mehrwertigen Phenolen im Bereich von 1 : 0 bis 1 : 10 liegt.

5. Verfahren nach den Ansprüchen 1 bis 4, **dadurch gekennzeichnet**, daß der pH-Wert der Reaktionsmischung im Bereich von 9 bis 12 liegt.

6. Verfahren nach den Ansprüchen 1 bis 5, **dadurch gekennzeichnet**, daß die Oxidation durch Behandlung mit Sauerstoff oder einem sauerstoffhaltigen Gas erfolgt.

7. Verfahren nach den Ansprüchen 1 bis 5, **dadurch gekennzeichnet**, daß die Oxidation elektrochemisch erfolgt.

8. Verfahren nach den Ansprüchen 1 bis 5, **dadurch gekennzeichnet**, daß die Oxidation plasmachemisch erfolgt.

9. Verfahren nach den Ansprüchen 1 bis 5, **dadurch gekennzeichnet**, daß die Oxidation durch milde Oxidationsmittel erfolgt.

10. Huminate, hergestellt nach einem Verfahren gemäß der Ansprüche 1 bis 9.

11. Verwendung der Huminate hergestellt nach einem der Ansprüche 1 bis 9, zur Herstellung von Mitteln zur Wundheilung.

12. Verwendung der Huminate, hergestellt nach einem der Ansprüche 1 bis 9, zur Herstellung von hochwirksamen synthetischen Moorbädern.

13. Verwendung der Huminate, hergestellt nach einem der Ansprüche 1 bis 9, zur Herstellung eines Mittels zur Behandlung gestreßter Fische.

14. Verwendung der Huminate, hergestellt nach einem der Ansprüche 1 bis 9, zur Detoxifizierung von Wasser.

15. Verwendung der Huminate, hergestellt nach einem der Ansprüche 1 bis 9, zur Detoxifizierung von Flächen und festen Gegenständen.

16. Verwendung der Huminate, hergestellt nach einem der Ansprüche 1 bis 9, zur Herstellung einer Nasenarznei.

17. Verwendung der Huminate, hergestellt nach einem der Ansprüche 1 bis 9, zur Herstellung eines Antischuppenshampoos.

## Claims

1. A process for preparing alkali or ammonium huminates, **characterized in that** alkaline or ammoniacal aqueous solutions, which contain compounds of the general formulae 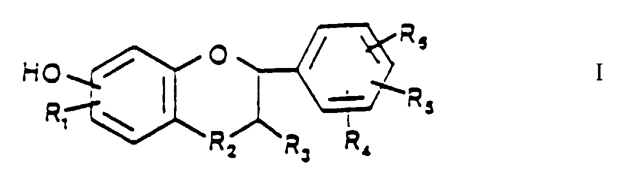 and/or 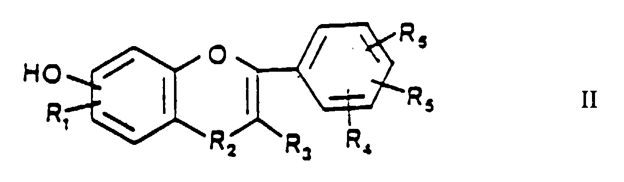 in which R₁, R₃ and R₄ are the same or different and represent an OH group or hydrogen, R₂ represents a CO or CH₂ group, and R₅ and R₆ are the same or different and represent hydrogen, an OH group or a methoxy group, wherein R₄, R₅ and R₆ are not all hydrogen at the same time, are oxidized at a temperature in the range of from 15 to 40°C, after that the reaction mixture is set and/or buffered to a pH value in the range of from 4·5 to 7·0 and the alkali or ammonium huminates with a molecular weight of up to 30,000 D are purified by preparative chromatography methods, ultrafiltration, ultracentrifugation or electrodialysis and are separated from undesired by-products.

2. A process according to Claim 1, **characterized in that** compounds of the general formulae I and/or II are used as pure substances or in any desired mixture with one another.

3. A process according to Claims 1 and 2, **characterized in that** compounds of the general formulae I and/or II are used mixed with multivalent phenols.

4. A process according to Claim 3, **characterized in that** the molar ratio of the compounds of the general formulae I and/or II to multivalent phenols is in the range of from 1 : 0 to to 1 : 10.

5. A process according to Claims 1 to 4, **characterized in that** the pH value of the reaction mixture is in the range of from 9 to 12.

6. A process according to Claims 1 to 5, **characterized in that** the oxidation is performed by treatment with oxygen or an oxygen-containing gas.

7. A process according to Claims I to 5, **characterized in that** the oxidation is performed electrochemically.

8. A process according to Claims 1 to 5, **characterized in that** the oxidation is performed plasmachemically.

9. A process according to Claims 1 to 5, **characterized in that** the oxidation is performed by mild oxidizing agents.

10. Huminates prepared by a process according to Claims 1 to 9.

11. Use of the huminates prepared by a process according to Claims 1 to 9, for preparing agents for healing wounds.

12. Use of the huminates prepared by a process according to Claims 1 to 9, for preparing highly effective synthetic mud baths.

13. Use of the huminates prepared by a process according to Claims 1 to 9, for preparing an agent for treating stressed fish.

14. Use of the huminates prepared by a process according to Claims I to 9, for detoxifying water.

15. Use of the huminates prepared by a process according to Claims 1 to 9, for detoxifying surfaces and solid objects.

16. Use of the huminates prepared by a process according to Claims 1 to 9, for preparing a nasal medicament.

17. Use of the huminates prepared by a process according to Claims 1 to 9, for preparing an anti-dandruff shampoo.

## Revendications

1. Procédé de préparation d'humates alcalins ou d'ammonium, caractérisé en ce que les solutions aqueuses alcalines ou ammoniacales contenant des composés de formules générales 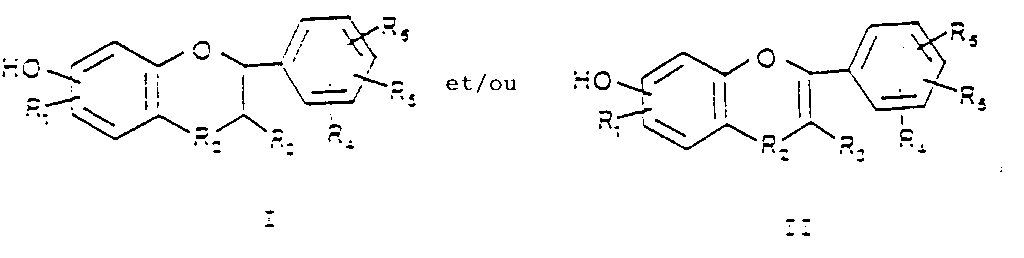 où R₁, R₃ et R₄ sont identiques ou différents et représentent un groupe OH ou l'hydrogéne, R₂ signifie un groupe CO ou CH₂ et R₅ et R₆ sont identiques ou différents et représentent l'hydrogéne, un groupe OH ou un groupe méthoxy, R₄, R₅ et R₆ n'étant pas tous en même temps de l'hydrogène, sont oxydés à une température se situant dans la gamme de 15 à 40°C, que le mélange réactionnel est ensuite ajusté et/ou tamponné à une valeur de pH dans la gamme de 4,5 à 7,0 et que les humates alcalins ou d'ammonium d'un poids moléculaire jusqu'à 30 000 D sont purifiés par des méthodes de chromatographie préparative. par ultrafiltration, ultracentrifugation ou électrodialyse et sont séparés des sous-produits indésirables.

2. Procédé selon la revendication 1, caractérisé en ce que des composés de formules générales I et/ou II sont mis en oeuvre sous forme de substances pures ou en mélange entre eux au choix.

3. Procédé selon les revendications 1 et 2, caractérisé en ce que des composés de formules générales I et/ou II sont mis en oeuvre en mélange avec des polyphénols.

4. Procédé selon la revendication 3, caractérisé en ce que le rapport molaire des composés de formules générales I et/ou II aux polyphénols se situe dans la gamme de 1:0 à 1:10.

5. Procédé selon les revendications 1 à 4, caractérisé en ce que la valeur de pH du mélange réactionnel se situe dans la gamme de 9 à 12.

6. Procédé selon les revendications 1 à 5, caractérisé en ce que l'oxydation s'opère par traitement à l'aide d'oxygène ou d'un gaz contenant de l'oxygène.

7. Procédé selon les revendications 1 à 5, caractérisé en ce que l'oxydation a lieu par voie électrochimique.

8. procédé selon les revendications 1 à 5, caractérisé en ce que l'oxydation a lieu par voie plasma-chimique.

9. Procédé selon les revendications 1 à 5, caractérisé en ce que l'oxydation s'opère avec des oxydants doux.

10. Humates, préparés selon un procédé conforme aux revendications 1 à 9.

11. Utilisation des humates, préparés selon l'une des revendications 1 à 9, pour la préparation d'agents pour la cicatrisation.

12. Utilisation des humates, préparés selon l'une des revendications 1 à 9, pour la préparation de bains de boue synthétiques hautement efficaces.

13. Utilisation des humates, préparés selon l'une des revendications 1 à 9, pour la préparation d'un agent pour le traitement de poissons stressés.

14. Utilisation des humates, préparés selon l'une des revendications 1 à 9, pour la détoxication de l'eau.

15. Utilisation des humates, préparés selon l'une des revendications 1 à 9, pour la détoxication de surfaces et d'objets solides.

16. Utilisation des humates, préparés selon l'une des revendications 1 à 9, pour la préparation d'un médicament nasal.

17. Utilisation des humates, préparés selon l'une des revendications 1 à 9, pour la préparation d'un shampooing antipelliculaire.
